# EUROPEAN PATENT SPECIFICATION

(11) **EP 2 939 656 B1**
(45) Date of publication and mention of the grant of the patent: **23.09.2020**
(21) Application number: 15165559.4
(22) Date of filing: 29.04.2015
(51) Int. Cl.: A61K 8/41, A61Q 5/10, A61K 8/73, A61K 8/81, A61K 8/06

(54) **AQUEOUS OXIDATIVE DYEING COMPOSITION**
WÄSSRIGE OXIDATIVE FÄRBEZUSAMMENSETZUNG
COMPOSITION DE TEINTURE OXYDANTE AQUEUSE

(30) Priority: 30.04.2014 EP 14166611
(43) Date of publication of application: 04.11.2015
(73) Proprietor: Kao Germany GmbH, 64297 Darmstadt (DE)
(72) Inventor: Leuwer, Somayeh Samy, 64297 Darmstadt (DE); Schmelz, Sandra, 64297 Darmstadt (DE); Kerbs Rabkin, Viktoria, 64297 Darmstadt (DE)

(56) References cited:
- EP-A1- 1 629 825
- EP-A1- 2 471 503
- WO-A2-2014/020147
- US-A1- 2004 103 488
- US-A1- 2004 131 576
- US-A1- 2006 000 032
- US-A1- 2006 293 213

## Description

The present invention relates to an aqueous oxidative hair dyeing composition comprising a cationic surfactant as an emulsifier, a cationic polymer with a high cationic charge density and a heteropolysaccharide gum.

Hair dyeing is a very ancient art and the demand for it is constantly increasing. The reasons for dyeing hair are different. Whereas some consumers want to hide grey hair, others just want to experiment new hair colours to look more attractive and to comply with their style. Besides the dyeing performance in terms of colour results and properties (for example intensity, homogeneity, grey coverage), it is very important to the consumer that the hair is left in a good condition with good cosmetic properties after a hair dyeing process, which is a chemical process that may damage the hair.

Despite the recent developments, there is still a need for further improvements especially in terms of reducing hair damage and leaving hair in a cosmetically acceptable status and at the same time preserving the dyeing performance. Hair cosmetic properties referred to are especially wet and dry combability, elasticity, natural softness and feeling upon touching.

Moreover, oxidative hair dyeing compositions in form of emulsions are particularly relevant and must be physically stable prior to use so that the dyes are uniformly distributed on the hair. Emulsifying surfactants are added to the emulsion to form and to keep it stable. Common emulsifiers in hair dyeing compositions are anionic or non-ionic surfactants. Non-ionic surfactants are particularly useful because they are compatible with various functional materials. Anionic emulsifiers are largely used in hair dyeing compositions due to their availability. At the same time, the use of cationic emulsifier is of great interest in hair dyeing composition because of their conditioning properties. It is a challenge to formulate a hair dyeing composition which leads to a good dyeing performance, has good rheological properties and is stable and at the same time leaves hair in a better cosmetic status.

The inventors of the present invention have surprisingly found out that a hair dyeing composition comprising a cationic surfactant, a cationic polymer with a high cationic charge density and a heteropolysaccharide gum, colours hair homogeneously with comparable dyeing performance as conventional ones but leaves the hair in a much better condition with good cosmetic properties in terms of softness and combability of wet and/or dry hair. Moreover, it has been found out that using the dyeing composition of the present invention, which is preferably free from anionic emulsifiers, prevents from the appearance of grey shimmering on the hair after the dyeing process. This effect is characterized by a light grey shimmering on various parts of the hair. Without being bound by the theory, it is believed that this can be due to the fact that previously used hair products such as for conditioning and/or styling hair based on cationic ingredients may have left some traces layers deposited onto hair (build-up effect), which may precipitate with anionic emulsifiers leading to a grey shimmering appearance of the parts, which were previously overloaded with cationic conditioning agents.
EP 1629825 discloses a tintig cream low charge density cationic polymer and not comprising cationic surfactant. US 2006/000032 is on agent for colouring hair not comprising any cationic surfactant. EP 2471503 is on dyeing composition which does not comprise high charge density cationic polymer. WO 2014/020147 is on dyeing composition comprising high charge density cationic polymer but not comprising cationic surfactant and heteropolysaccharide. In US 2004103488 use of cationic polymers are not foreseen. US 2004/131576 is on dyeing composition lacking heteropolysaccharide.

The first object of the invention is an aqueous composition for dyeing hair comprising at least one oxidation hair dye precursor, at least one cationic surfactant, at least one cationic polymer with a cationic charge density of greater than or equal to 4 meq/g at a total concentration of from 0.05 and 2%, by weight calculated to the total of the composition and at least one heteropolysaccharide gum it is free from anionic emulsifiers.
The second object of the invention is on a process for dyeing hair wherein the composition of the present invention is mixed with an aqueous composition comprising an oxidizing agent prior to application, the mixture is applied onto hair and rinsed off from hair after processing for 1 to 45 min, optionally hair is shampooed and dried.
Another object of the invention is a kit for dyeing hair which comprises an individually packaged first component comprising the aqueous composition of the present invention and an individually packaged second aqueous composition comprising an oxidizing agent.

Composition of the present invention is an oil-in-water emulsion comprising water in the range of 50 to 90%, more preferably in the range of 55 to 85%, most preferably in the range of 60 to 80% by weight, calculated to the total composition.

The composition comprises at least one oxidation dye precursor. Suitable ones are tetraaminopyrimidines, in particular 2,4,5,6-tetraaminopyrimidine and the lower alkyl derivatives thereof; suitable triaminohydroxypyrimidines are, for example 4-hydroxy-2,5,6-triaminopyrimidine, 2-hydroxy-4,5,6-triaminopyrimidine and 5-hydroxy-2,4,6-triaminopyrimidine; suitable mono and diamino dihydroxypyrimidines are, for example, 2,6-dihydroxy-4,5-diaminopyrimidine, 2,4-diamino-6-hydroxy-pyrimidine or 4,6-dihydroxy-2,5-diaminopyrimidine or the water-soluble salts thereof, aminophenol derivatives such as 4-aminophenol, 4-amino-3-methylphenol, 2-chloro-4-aminophenol, 2,6-dichloro-4-aminophenol, 2,4-diamino-phenol, 2,6-dibromo-4-aminophenol and/or 2-aminophenol and water-soluble salts thereof, furthermore, phenylenedimanine derivatives such as 2,5-diamino-toluene, 2-n-propyl or 2-ethyl-p-phenylenediamine, 2,6-dimethyl-p-phenylenediamine, 2-(2,5-diaminophenyl) ethanol, 1-amino -4-bis-(2'-hydroxy-ethyl)aminobenzene, 2-(2-hydroxyethyl amino)-5-aminotoluene, 4,4'-diaminodiphenylamine, 4-aminodiphenylamine, 2-amino-5-N,N-diethyl aminotoluene, 4-amino-N-ethyl-N-isopropylaniline, 2-chloro-p-phenylenediamine, 1-l3-hydroxyethyl-2,5-diamino-4-chlorobenzene, 1-13-hydroxyethyl-2,5-diamino-4-methyl benzene, 2-methoxy-p-phenylenediamine, N, N-diethyl-p-phenylenediamine, 1-amino-4-l3-methoxyethyl aminobenzene, 1-dimethyl-amino-4-aminobenzene, 1-hydroxy-2,5-diamino-4-methyl benzene, 1-hydroxymethyl-2,5-diaminobenzene, 1,3-dimethyl-2,5-diaminobenzene, 1 ,4-diamino isopropyl benzene and/or 1-amino-4-∼-hydroxypropyl aminobenzene or the water-soluble salts thereof, pyrazole derivatives such as 1-hydroxyethyl-4,5-diaminopyrazole, 3,4-diamino-5-hydroxypyrazole, 3,5-diaminopyrazole, 3,5-diaminopyrazol-1-carboxamide, 3-amino-5-hydroxypyrazole, 1-phenyl-2-methylpyrazole, 1-phenyl-3-methylpyrazole-5-one, 3,5-dimethylpyrazole,3,5-dimethylpyrazole-1-methanol, 3,5-dimethylpyrazole,3,5-dimethylpyrazole-1-methanol, 1-methyl-4,5-diaminopyrazole, 1-methylethyl-4,5-diaminopyrazole, 1-phenylmethyl-4,5-diaminopyrazole, 1-methyl-4 ,5-diaminopyrazole, 1-(4-methylphenyl)methyl-4,5-diaminopyrazole, 1-methyl-3-phenyl-4,5-diaminopyrazole and the water-soluble salts.
The composition according to the invention optionally but preferably comprises at least one coupling substance, which can be selected from resorcinol, 2-methyl resorcinol, 4-chlororesorcinol, 2-amino-4-chlorophenol, 5-amino-4-methoxy-2-methylphenol, 3-amino-phenol, 1-methyl-2-hydroxy-4-aminobenzene, 3-N ,N-dimethyl aminophenol, 2.6-dihydroxy-3.5-dimethoxypyridine, 5-amino-3-methylphenol, 6-amino-3-methylphenol, 3-amino-2-methylamino-6-methoxypyridine, 2-amino-3-hydroxy-pyridine, 2-dimethyl-amino-5-aminopyridine, 2,6-diaminopyridine, 1,3-diaminobenzene, 1-amino-3-(2'-hy-droxyethylamino)benzene, 1-amino-3-[bis(2'-hydroxy-ethyl) amino]benzene, a-naphthol, 4,6-dichlororesorcinol, 1,3-diamino-toluene, 1-hydroxy naphthalene, 4-hydroxy-1 ,2-methylenedioxy benzene, 1 ,5-dihydroxy naphthalene, 1 ,6-dihydroxy naphthalene, 1 ,7-dihydroxy naphthalene, 2,7-dihydroxy naphthalene, 1-hydroxy-2-methyl naphthalene, 4-hydroxy-1.2-methyldioxy benzene, 2,4-diamino-3-chlorophenol, 5-amino-2-methoxyphenol and/or 1-methoxy-2-amino-4-(2' -hydroxyethyl amino)benzene or the water-soluble salts thereof.

The above presented lists of oxidative dye precursors and coupling substances shall not exclude the addition of further developing and coupling substances which are not namely mentioned.

The weight proportion of the developing substances to the coupling substances ranges in general between 1 : 8 to 8 : 1, preferably 1 : 5 to 5 :1, in particular 1 : 2 to 2 : 1.

According to the invention, the oxidative dye composition comprises oxidative hair dyes at a total concentration of 0.001 to 15%, preferably 0.01 to 12.5%, more preferably 0.05 to 10%, most preferably 0.1 to 7.5% by weight calculated to total composition.

The composition of the present invention may furthermore comprise hair direct dyes of neutral, cationic and anionic character. Examples of suitable cationic dyes are Basic Blue 6, Basic Blue 7, Basic Blue 9, Basic Blue 26, Basic Blue 41, Basic Blue 99, Basic Brown 4, Basic Brown 16, Basic Brown 17, Natural Brown 7, Basic Green 1, Basic Red 2, Basic Red 12, Basic Red 22, Basic Red 51, Basic Red 76, Basic Violet 1, Basic Violet 2, Basic Violet 3, Basic Violet 10, Basic Violet 14, Basic Yellow 57, Basic yellow 87 and Basic orange 31. According to the invention, suitable cationic dyestuffs are in principal those any available on the market for cosmetic hair colouring applications. For this purpose, special reference is made to the PCT application WO 95/15144 of Ciba-Geigy AG. The content of the PCT application WO 95/15144 is by reference incorporated here.

Examples of suitable direct anionic dyes are Acid Black 1, Acid Blue 1, Acid Blue 3, Food Blue 5, Acid Blue 7, Acid Blue 9, Acid Blue 74, Acid Orange 3, Acid Orange 6, Acid Orange 7, Acid Orange 10, Acid Red 1, Acid Red 14, Acid Red 18, Acid Red 27, Acid Red 50, Acid Red 52, Acid Red 73, Acid Red 87, Acid Red 88, Acid Red 92, Acid Red 155, Acid Red 180, Acid Violet 9, Acid Violet 43, Acid Violet 49, Acid Yellow 1, Acid Yellow 23, Acid Yellow 3, Food Yellow No.8, D&C Brown No.1, D&C Green No.5, D&C Green No.8, D&C Orange No.4, D&C Orange No. 10, D&C Orange No. 11, D&C Red No. 21, D&C Red No. 27, D&C Red No. 33, D&C Violet 2, D&C Yellow No.7, D&C Yellow No.8, D&C Yellow No. 10, FD&C Red 2, FD&C Red 40, FD&C Red No.4, FD&C Yellow No.6, FD&C Blue 1, FoodBlack 1, Food Black 2, Disperse Black 9 and Disperse Violet 1 and their alkali metal salts such as sodium, potassium.

Additional suitable direct dyes are of the following chemical structures.

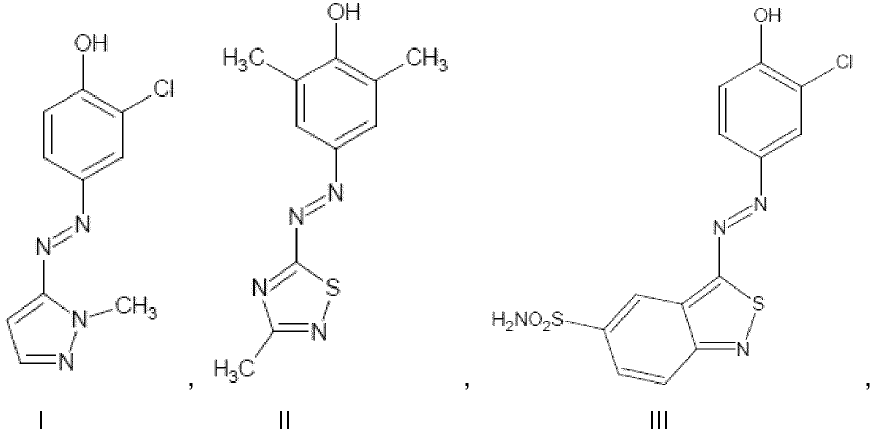

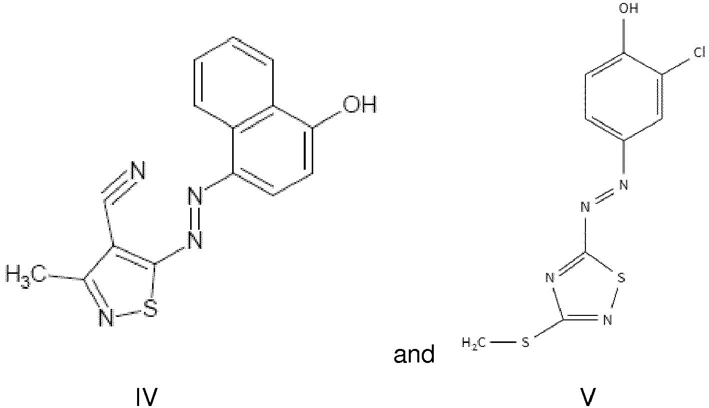

Some examples to those suitable neutral dyes (HC dyes), so called nitro dyes, are He Blue No.2, He Blue NoA, He Blue No.5, He Blue No.6, He Blue No.7, He Blue No.8, He Blue No.9, He Blue No.1 0, He Blue No.11, He Blue No.12, He Blue No.13, He Brown No.1, He Brown No.2, He Green No.1, He Orange No.1, He Orange No.2, He Orange No.3, He Orange No.5, He Red BN, He Red No.1, He Red No.3, He Red No.7, He Red No.8, He Red No.9, He Red No.10, He Red No.11, He Red No.13, He Red No.54, He Red No.14, He Violet BS, He Violet No.1, He Violet No.2, He Yellow No.2, He Yellow NoA, He Yellow No.5, He Yellow No.6, He Yellow No.7, He Yellow No.8, He Yellow No.9, He Yellow No.10, He Yellow No.11, He Yellow No.12, He Yellow No.13, He Yellow No.14, He Yellow No.15, 2-Amino-6-chloro-4-nitrophenol, picramic acid, 1,2-Diamino-4-nitrobenzol, 1,4-Diamino-2-nitrobenzol, 3-Nitro-4-aminophenol, 1-Hydroxy-2-amino-3-nitrobenzol and 2-hydroxyethylpicramic acid.

Plant dyestuffs can also be used alone or in combination with synthetic direct-acting dyestuffs, for example henna (red or black), alkanna root, laccaic acid, indigo, logwood powder, madder root and rhubarb powder, etc.

The composition of the present invention comprises at least one cationic surfactant as an emulsifier. Suitable and preferred ones are mono alkyl quaternary ammonium surfactants. With the term mono alkyl it is meant that quaternary ammonium surfactant includes only one alkyl chain which has more than 8 C atoms. Preferably at least one mono alkyl quaternary ammonium surfactant is selected from the compounds with the general formula 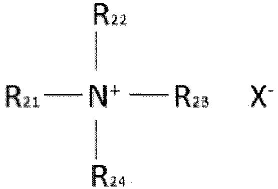 where R₂₁ is saturated or unsaturated, branched or non-branched alkyl chain with 8-22 C atoms or R_{2S}CONH(CH₂)n or R_{2S}COO(CH₂)n, where R₂₅ is saturated or unsaturated, branched or non-branched alkyl chain with 7-21 C atoms and n has typical value of 1 -4, and R₂₂, R₂₃ and R₂₄ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

Suitable cationic emulsifiers are, for example, long-chain quaternary ammonium compounds which can be used alone or in admixture with one another, such as cetyl trimethyl ammonium chloride, myristoyl trimethyl ammonium chloride, behentrimonium chloride, trimethyl cetyl ammonium bromide, stearyltrimethyl ammonium chloride, steartrimonium chloride, stearamidopropyltrimethylammonium chloride, stearamidopropyltrimonuim chloride.

Cationic surfactants are included into the compositions at a total concentration of 0.1 to 10%, preferably 0.2 to 7.5%, more preferably 0.25 to 5% and most preferably 0.5 to 3% by weight, calculated to the total composition.

The composition of the invention comprises at least one cationic polymer with a cationic charge density of greater than or equal to 4 milliequivalens per gram (meq/g), preferably greater than or equal to 5 meq/g and most preferably greater than or equal to 6 meq/g. Cationic charge density may be calculated as the number of cationic charges per unit divided by molecular weight of the unit and multiplied by 1000 in order to express it as meq/g.

Suitable cationic polymers include for example dimethyldiallylammonium chloride homopolymers and polymeric quaternary ammonium salt formed from methylvinylimidazolium chloride and vinylpyrrolidone, respectively referred to in the industry (CTFA) as Polyquaternium-6 and Polyquaternium-16.

Concentration of cationic polymer in the compositions is in the range of 0.05 and 2%, preferably 0.05 to 1.5%, more preferably 0.1 to 1 % by weight calculated to the total of the composition

The composition of the invention comprises at least one heteropolysaccharide gum. Suitable heteropolysaccharide gums are those having a thickening effect in the compositions as disclosed above. Suitable heteropolysaccharide gums are, for example, xanthan gum, carrageenan, guar gum, alginate or locust bean gum. Xanthan gum is the preferred heteropolysaccharide gum in the present invention.

The concentration of the heteropolysaccharide gum is in the range of 0.01 to 1.5%, preferably from 0.02 to 1.25%, more preferably from 0.03 to 1.0% and most preferably from 0.1 to 0.75% by weight, calculated to the total of the composition.

The composition of the present invention is mixed with an aqueous composition comprising at least one oxidizing agent. The oxidizing agents suitable are hydrogen peroxide, urea peroxide, melamine peroxide or perborate salts. The preferred oxidizing agent is hydrogen peroxide, preferably at a concentration in a range of 2 to 12 % by weight calculated to the total composition.

The kit of above preferably comprises an individually packaged first component comprising an oxidizing agent and an individually packaged second component comprising the at least one oxidation dye. The pH of the composition comprising at least one oxidizing agent is in the range of 2 to 5, preferably 2.5 to 4 and more preferably 3 to 4. The composition of the present invention have a pH in the range of 6 to 12 and preferably 6.5 to 11, more preferably 6.8 to 11 and most preferably 8 to 10.5 and in particular 9 to 10. The compositions after mixing have a pH in the range of 6.8 to 11 and most preferably 8 to 10.5 and in particular 9 to 10.

The composition comprises at least one alkalizing agent. Suitable alkalizing agents in the present invention are ammonia and compounds according to the general formula

R₁₁R₁₂R₁₃N

wherein R₁₁, R₁₂ and R₁₃ are same or different H, C₁-C₆ alkyl, C₁-C₆ monohydroxyalkyl or C₂-C₆ polyhydroxyalkyl with the condition that at least one of R₁₁, R₁₂ and R₁₃ is a mono or polyhydroxyalkyl. Preferably R₁₁, R₁₂ and R₁₃ are same or different H, C₁-C₄ alkyl, C₁-C₄ monohydroxyalkyl or C₂-C₄ polyhydroxyalkyl with the condition that at least one of R₁₁, R₁₂ and R₁₃ is a mono or polyhydroxyalkyl.

Suitable alkanolamines according to the general formula of above are monoethanolamine, diethanolamine, triethanolamine, monoethanol methylamine, monoethanoldimethylamine, diethanolmethylamine, monoethanolethylamine, monoethanoldiethylamine, diethanolethylamine, monoethanolpropylamine, monoethanoldipropylamine, diethanolpropylamine, monoethanolbutylamine and diethanolbutylamine.

Preferred alkanolamines are monoethanolamine, diethanolamine and triethanolamine. The most preferred is monoethanolamine.

Within the meaning of the present invention it should also be understood that oxidative dyeing compositions and/or ready to use oxidative dyeing compositions can comprise more than one alkanolamine such as a mixture of two or three alkanolamines.

The concentration of at least one alkalizing agent varies between 1 and 35%, preferably 1 and 30, more preferably 2.5 and 25 and most preferably 2.5 to 20% by weight calculated to the total composition.

The composition of the present invention preferably comprises one or more fatty alcohol, preferably of the general formula

R₃₁OH

wherein R₃₁ is a linear or branched, saturated or unsaturated alkyl chain with 12 to 22 C atoms. Suitable fatty alcohols are myristyl alcohol, cetyl alcohol, stearyl alcohol and behenyl alcohol and their mixtures. Most preferred is the mixture of cetyl and stearyl alcohols also known as cetearyl alcohol.

The concentration of one or more fatty alcohols is in the range of 1 to 25%, preferably 2 to 20%, more preferably 2.5 to 15% and most preferably 5 to 12.5% by weight calculated to the total composition.

The composition of the present invention may comprise one or more non-ionic surfactant as emulsifier. Suitable non-ionic surfactants are alkyl polyglucosides of the general formula

R₄₁-0-(R₄₂0)nO-Zₓ

wherein R₄₁ is an alkyl group with 8 to 18 carbon atoms, R₄₂ is an ethylene or propylene group, Z is a saccharide group with 5 to 6 carbon atoms, n is a number from 0 to 10 and x is a number between 1 and 5. Examples are decyl polyglucoside and cocoyl polyglucoside, both being commercially available.

Further non-ionic surfactant components are, for example, long-chain fatty acid mono-and dialkanolamides, such as coco fatty acid monoethanolamide and myristic fatty acid monoethanolamide.

Further additionally useful non-ionic surfactants are, for example, the various sorbitan esters, such as polyethylene glycol sorbitan stearic acid ester, fatty acid polyglycol esters or poly-condensates of ethyleneoxide and propyleneoxide, as they are on the market, for example, under the trade name "PluronicsR".

Further non-ionic surfactants as emulsifiers useful in the compositions according to invention are C1-22-fatty alcohol ethoxylates. Especially suited are C1-C22-fatty alcohol ethers, the alkyl polyglycol ethers known by the generic terms "Laureth", "Myristeth", "Oleth", "Ceteth", "Oeceth","Steareth" and "Ceteareth" according to the CTFA nomenclature, including addition of the number of ethylene oxide molecules, e.g., "Laureth-16": J The average degree of ethoxylation thereby ranges between 1 and 30.

Nonionic emulsifiers are preferably used at a total concentration of 0.1 to 15%, preferably to 0.5 to 10% and most preferably from 1 to 5% by weight calculated to the total composition.

The composition of the present invention may comprise allantoin and/or its derivative to improve mildness of the composition on the scalp. Suitable are allantoin, allantoin acetyl methionine, allantoin ascorbate, allantoin biotine, allantoin calcium pantothenate, allantoin galacturonic acid and its salts, allantoin glycyrrhetinic acid and its salts, allantoin PABA, alantoin panthenol, allantoin polygalacturonic acid and its salts and allantoin PCA and its salts.

Preferred are allantoin, allantoin acetyl methionine, allantoin ascorbate, allantoin biotine, allantoin PABA, allantoin panthenol and allantoin PCA and its salts. More preferably the allantoin derivative is selected from compounds of allantoin acetyl methionine, allantoin ascorbate, allantoin PABA and alantoin panthenol. Most preferred compounds are allantoin, allantoin acetyl methionine, allantoin ascorbate, allantoin PABA and alantoin panthenol. Particularly preferred is allantoin.

The concentration of allantoin and/or its derivative may be in the range of 0.001 to 5%, preferably 0.01 to 4%, more preferably 0.05 to 3% and most preferably 0.1 to 2.5% and in particular 0.2 to 1.5% by weight calculated to the total composition.

The composition of the present invention preferably comprises oil, more preferably natural oil and most preferably natural triglycerides.

Concentration of oil varies between 0.1 and 25%, preferably 0.5 and 25% and more preferably 1 and 20%, most preferably 2 and 15%, in particular 2.5 and 10% by weight calculated to the total composition.

Suitable natural oils are paraffin oils. Suitable natural triglycerides are argan oil, shea butter oil, karite oil, olive oil, almond oil, avocado oil, ricinus oil, coconut oil, palm oil, sesame oil, peanut oil, sunflower oil, peach kernel oil, wheat germ oil, macadamia nut oil, macadamia oil, night primrose oil, jojoba oil, castor oil, soya oil, lanolin, passiflora oil, black cumin oil, borage oils, grapeseed oil, hempseed oil, kukui nut oil, and rosehip oil. Preferred are argan oil, shea butter oil, karite oil, olive oil, almond oil, avocado oil, ricinus oil, coconut oil, palm oil, sesame oil, peanut oil, whale oil, sunflower oil, peach kernel oil, wheat germ oil, macadamia nut oil, macadamia oil, night primrose oil, jojoba oil, castor oil, and soya oil. More preferred are argan oil, shea butter oil, karite oil, macadamia nut oil, macadamia oil, olive oil, almond oil, avocado oil, ricinus oil, coconut oil, palm oil, sesame oil, peanut oil, sunflower oil, peach kernel oil, wheatgerm oil, jojoba oil, castor oil, and soya oil. Most preferred are argan oil, shea butter oil, karite oil, olive oil, almond oil, avocado oil, coconut oil, macadamia nut oil, macadamia oil, palm oil, sesame oil, peach kernel oil, wheatgerm oil, jojoba oil, and soya oil. Particularly preferred are argan oil, shea butter oil and karite oil which may be comprised as a single oil component or in admixture with each other.

Further suitable oil components are synthetic oils such as silicones known with CTFA adopted name dimethicone, cyclomethicone, and ary/ates silicones such as phenyl trimethicone which are available commercially from Dow Corning.

Further suitable synthetic oils are fatty acid fatty alcohol esters such as octyl palmitate, isocetyl palmitate, isopropyl palmitate and octyl stearate.

The composition of the present invention preferably comprises at least one diamine compound. Preferred diamide compounds are those according to the general structure 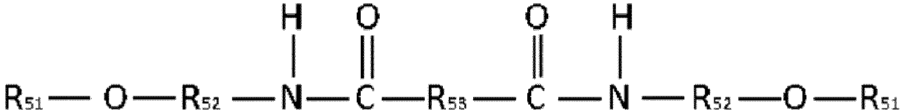 wherein R₅₁ is a linear or branched, saturated or unsaturated alkyl chain with 1 to 12 C atoms which may be substituted with hydroxy and/or alkoxy groups, preferably R₅₁ is linear or branched, saturated or unsaturated alkyl chain with 1 to 12 C atoms which may be substituted by 1 to 3 substituents selected from a hydroxy group and C 1 to C6 alkoxy group, more preferably R₅₁ is a unsubstituted alkyl group with 1 to 12 C atoms, and alkyl group with 2 to 12 C atoms substituted by one or two hydroxyl groups, by one alkoxy group with 1 to 6 C atoms or by one hydroxyl and one alkoxy group with 2 to 6 C atoms, R₅₂ is linear or branched alkyl chain with 1 to 5 C atoms, preferably linear or branched alkyl chain with 2 to 5 C atoms and more preferably an alkyl chain with 2 to 3 C atoms, and R₅₃ linear or branched, saturated or unsaturated alkyl chain with 1 to 22 C atoms, preferably linear or branched, saturated or unsaturated alkyl chain with 11 to 22 C atoms.

Preferred individual diamide compounds are the ones according to the following formula A to G

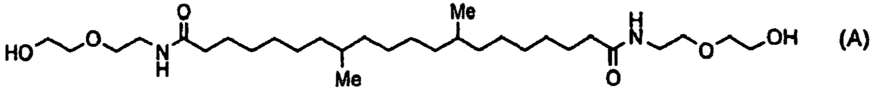

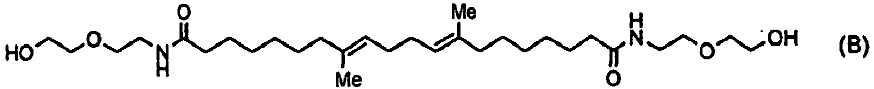

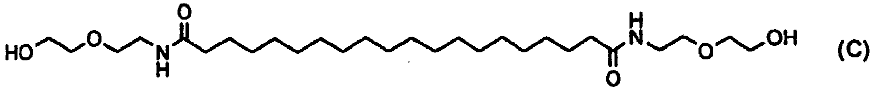

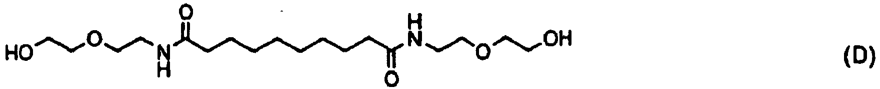

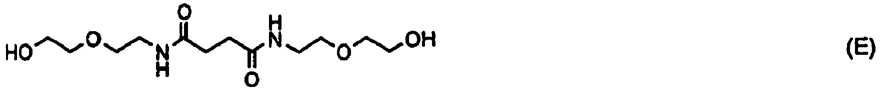

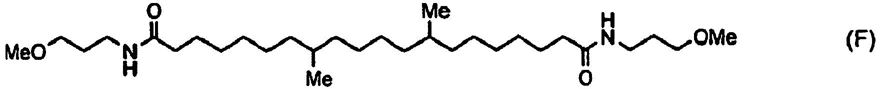

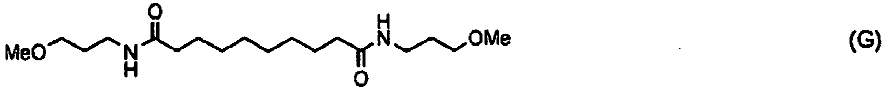

Particularly preferred diamide compound is the compound F, which is bis (methoxypropylamido) isodocosane and commercially available from Kao Corporation -Japan.

Concentration of diamide compound is in the range of 0.001 to 5%, preferably 0.002 to 3% more preferably 0.005 to 2% and most preferably 0.01 to 1 % by weight calculated to total composition.

Compositions of the present invention may comprise further at least one compound according to the formula 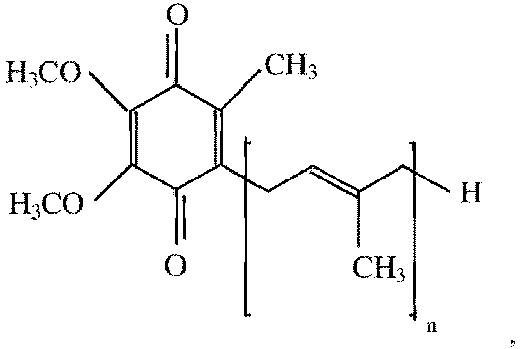 where n is a number from 1 to 10.

The compounds of the above formula are known as Ubiquinone, and also are known as Coenzyme. It should be noted that the compositions of the present invention can certainly comprise more than one ubiquinone. Preferred ubiquinones are the ones where n is a number between 6 and 10 and especially preferred is Ubiquinone 50 where n is 10, also known as Coenzyme Q10. Concentration ubiquinone of the above formula in the compositions is from 0.0001 to 1 %, preferably from 0.0002 to 0.75%, more preferably from 0.0002 to 0.5% and most preferably from 0.0005 to 0.5% by weight, calculated to total composition.

The composition of the present invention may comprise one or more polyols at a concentration of 0.1 to 5%, preferably 0.2 to 2.5% and more preferably 0.3 to 1.5% by weight calculated to the total concentration. The most preferred ones are panthenol, glycerin, propylene glycols, butylene glycols, propanediols and hexylene glycols.

The composition of the present invention may contain another component that is normally used in cosmetic ingredient in addition to the above components. Examples of such optional components include a preservative, a chelating agent, a stabilizer, an antioxidant, vitamins, preservative, a chelating agent, a stabilizer, an antioxidant, vitamins, fragrance.
The following examples are to illustrate the invention without limiting it.

### Example 1

**Table 1**

| | % by weight of active substance |
|---|---|
| Cetearyl alcohol | 8.0 |
| Cetrimonium Chloride | 1.0 |
| Polyquaternium-6* | 0.5 |
| Xantham Gum | 0.2 |
| 3,5-diamino-1-hydroxyethyl-pyrazol | 1.0 |
| Resorcinol | 0.5 |
| Monoethanolamine | 7.0 |
| Water | q.s. to 100 |
| *available under the trade name Genamin PDAC from Clariant | |

The above composition was prepared by dissolving and mixing the given components in water. The composition had a pH of 11.0

The above composition was mixed with a composition comprising 6% by weight of hydrogen peroxide at a weight ratio of 1:1 and applied onto the hair and processed for 30 min at ambient temperature. The composition was then rinsed off and the hair was dried.

As the result, the hair was homogeneously colored and at the same time, the hair cosmetic properties in terms of combability, elasticity, natural softness and feeling upon touching were very satisfying. Moreover, no grey shimmering effect was observed.

### Example 2

**Table 2**

| | % by weight of active substance |
|---|---|
| Cetearyl alcohol | 8.0 |
| Paraffinum liquidum | 1.5 |
| Behentrimonium chloride | 0.5 |
| Polyquaternium-6* | 0.5 |
| Xantham Gum | 0.1 |
| Allantoin | 0.7 |
| 3,5-diamino-1-hydroxyethyl-pyrazol | 1.0 |
| Resorcinol | 0.5 |
| Ammonia 25% | 8.0 |
| Water | q.s. to 100 |
| *available under the trade name Genamin PDAC from Clariant | |

The above composition was prepared by dissolving and mixing the given components in water. The composition had a pH of 11.

The above composition was mixed with a composition comprising 6% by weight of hydrogen peroxide at a weight ratio of 1:1 and applied onto the hair and processed for 30 min at ambient temperature. The composition was then rinsed off and the hair was dried.

As the result, the hair was homogeneously colored and at the same time, the hair cosmetic properties in terms of combability, elasticity, natural softness and feeling upon touching were very satisfying and no grey shimmering effect was observed.

### Example 3

**Table 3**

| | % by weight of active substance |
|---|---|
| Cetearyl alcohol | 8.0 |
| Ceteareth-2 | 2.0 |
| Cetrimonium chloride | 0.7 |
| Polyquaternium-6* | 0.4 |
| Xantham Gum | 0.1 |
| Argan Oil | 0.2 |
| Basic Red 51 | 0.2 |
| p-phenylenediamine | 0.5 |
| Resorcinol | 0.2 |
| HC Blue 17 | 0.1 |
| Basic Yellow 87 | 0.05 |
| Monoethanolamine | 4.0 |
| Ammonia 25% | 4.0 |
| Water | q.s. to 100 |
| *available under the trade name Genamin PDAC from Clariant | |

The above composition was prepared by dissolving and mixing the given components in water. The composition had a pH of 11.

The above composition was mixed with a composition comprising 6% by weight of hydrogen peroxide at a weight ratio of 1:1 and applied onto the hair and processed for 30 min at ambient temperature. The composition was then rinsed off and the hair was dried.

As the result, the hair was homogeneously colored and at the same time, the hair cosmetic properties in terms of combability, elasticity, natural softness and feeling upon touching were very satisfying and no grey shimmering effect was observed.

### Comparative examples

**Table 4**

| | Composition A (invention) | Composition B (comperative) | Composition C (comperative) |
|---|---|---|---|
| Cetearyl alcohol | 8.0 | 8.0 | 8.0 |
| Ceteareth-2 | 2.0 | 2.0 | 2.0 |
| Cetrimonium chloride | 0.5 | 0.5 | 0.5 |
| Sodium lauryl sulfate | - | 1.0 | - |
| Polyquaternium-6* | 0.5 | 0.5 | - |
| Polyquaternium-7* | - | - | 0.5 |
| Xantham Gum | 0.1 | 0.1 | 0.1 |
| 3,5-diamino-1-hydroxyethyl-pyrazol | 1.0 | 1.0 | 1.0 |
| Resorcinol | 0.5 | 0.5 | 0.5 |
| Monoethanolamine | 7.0 | 7.0 | 7.0 |
| Water | q.s. to 100 | q.s. to 100 | q.s. to 100 |
| * available under the trade name Merquat 100 from Lubrizol | | | |
| ** available under the trade name Merquat 550 from Lubrizol | | | |

The above composition was prepared by dissolving and mixing the given components in water. The composition had a pH of 11.

Composition A corresponds to a hair dyeing composition according to the present invention. Composition B, which comprises an anionic emulsifier, does not correspond to a composition according to the invention. Composition C comprises a polyquaternium-7 available under the trade name Merquat 550. Polyquaternium-7 is a polymeric polymeric quaternary ammonium salt of acrylamide and dimethyl diallyl ammonium chloride. Its cationic charge density depends on the mole/weight proportion of the monomer units. In the case of the Polyquaternium-7 with the tradename Merquat 550, the cationic charge density is 3.1 *meq*/*g.*
Properties of the compositions A, B and C were compared using half-head tests. In a first test, compositions A and B were tested against each other in a half-head test with 10 test persons having shoulder length damaged hair.

The procedure of the test was the following: composition A and B were mixed with a composition comprising 6% by weight of hydrogen peroxide at a weight ratio of 1:1 and an equal amount of each mixture was applied onto the hair and processed for 30 min at ambient temperature. The composition was then rinsed off and the hair was dried.

Visual and sensory evaluations of hair properties were then performed by hair dressers to evaluate the hair dyeing performance and the cosmetic properties of the hair. It was observed that the three compositions lead to a similar color result in term of colour direction and intensity.

Following observations were made concerning the appearance of a grey shimmering and reported in Table 5. The numbers in Table 5 represent the number of consumers out of 10, for which the presence of a grey shimmering on the hair was observed.

**Table 5**

| | Composition A | Composition B |
|---|---|---|
| Presence of grey shimmering | 0/10 | 7/10 |

Grey shimmering effects were more often observed when using a hair colouring composition based on composition B. It was also noticed that this effect was stronger on consumers, who normally use lots of care and styling products.

Following observations were made concerning the cosmetic properties of the hair in terms of wet and dry combability and softness of the hair and reported in Table 6. Wet and dry combability as well as softness of wet and dried hair were evaluated by professional hair dressers by slipping a hair strand from root to tip through their finger and by repeating this procedure 5 times. The numbers in Table 6 represent the number of consumers, for which the concerned property was preferred.

**Table 6**

| | Composition A | Compostion B | No preference |
|---|---|---|---|
| Wet combability | 8 | 1 | 1 |
| Dry combability | 7 | 1 | 2 |
| Softness of wet hair | 9 | 0 | 1 |
| Softness of fry hair | 7 | 0 | 3 |

The same procedure as described above was performed for the comparison of compositions of A and C. It was also observed that the three compositions lead to a similar colour result in term of colour direction and intensity. Following observations were made concerning the appearance of a grey shimmering (Table 7), the wet and dry combability and the softness of the hair (Table 8).

**Table 7**

| | Composition A | Composition C |
|---|---|---|
| Presence of grey shimmering | 0/10 | 4/10 |

**Table 8**

| | Composition A | Compostion C | No preference |
|---|---|---|---|
| Wet combability | 9 | 0 | 1 |
| Dry combability | 7 | 0 | 3 |
| Softness of wet hair | 8 | 0 | 2 |
| Softness of fry hair | 8 | 1 | 1 |

From the above results, it is clear that the hair dyeing composition according to the present invention presents some advantages in terms of hair cosmetic properties towards known compositions and at the same time lead to a satisfying hair colouring performance.

## Claims

1. Aqueous composition for dyeing hair **characterized in that** it comprises at least one oxidation hair dye precursor, at least one cationic surfactant as an emulsifier, at least one cationic polymer with a cationic charge density of greater than or equal to 4 meq/g at a total concentration of from 0.05 and 2%, by weight calculated to the total of the composition, at least one heteropolysaccharide gum and it is free from anionic emulsifiers.

2. Composition according to claim 1 **characterized in that** the composition is an oil-in-water emulsion and comprises water in the range of 50 to 90%, more preferably in the range of 55 to 85%, most preferably in the range of 60 to 80% by weight, calculated to the total composition.

3. Composition according to claims 1 and 2 **characterized in that** the at least one cationic surfactant is chosen from quaternary ammonium compounds according to general structure 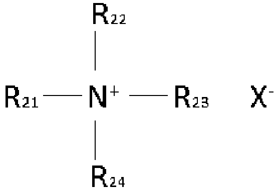 where R₂₁ is saturated or unsaturated, branched or non-branched alkyl chain with 8-22 C atoms or R₂₅CONH(CH₂)n or R₂₅COO(CH₂)n, where R₂₅ is saturated or unsaturated, branched or non-branched alkyl chain with 7-21 C atoms and n has typical value of 1 -4, and R₂₂, R₂₃ and R₂₄ are independent from each other lower alkyl chain with 1 to 4 carbon atoms, hydroxyl alky chain with 1 to 4 C atoms, or ethoxy or propoxy group with number of ethoxy or propoxy groups varying in the range of 1 to 4, and X is chloride, bromide or methosulfate.

4. Composition according to any of the preceding claims **characterized in that** the at least one cationic polymer with a cationic charge density of greater than or equal to 4 meq/g is chosen from polymeric dimethyl diallyl ammonium chloride.

5. Composition according to any of the preceding claims **characterized in that** the at least one heteropolysaccharide gum is chosen from xanthan gum, carrageenan, guar gum and lotus bean gum, preferably the heteropolysaccharide gum is chosen from xanthan gum and preferably present at a concentration of 0.1 to 2% by weight, calculated to the total of the composition.

6. Composition according to any of the preceding claims **characterized in that** the at least one cationic surfactant as an emulsifier is used at a total concentration of from 0.1 to 2% by weight, calculated to the total of the composition.

7. Composition according to any of the preceding claims **characterized in that** the cationic polymer with a cationic charge density of greater than or equal to 4 meq/g is used at a total concentration of from 0.05 to 1.5%, more preferably 0.1 to 1 % by weight, calculated to the total of the composition.

8. Composition according to any of the preceding claims **characterized in that** it comprises at least one alkalizing agent chosen from ammonia and compounds of the following general structure
R₁₁R₁₂R₁₃N
wherein R₁₁, R₁₂ and R₁₃ are same or different H, C₁-C₆ alkyl, C₁-C₆ monohydroxyalkyl or C₂-C₆ polyhydroxyalkyl with the condition that at least one of R₁₁, R₁₂ and R₁₃ is a mono or polyhydroxyalkyl.

9. Composition according to any of the preceding claims **characterized in that** the at least one alkalizing agent is chosen from alkanolamines, preferably at a concentration in the range of 1 to 35% by weight calculated to the total composition.

10. Composition according to any of the preceding claims **characterized in that** it additionally comprises a hair direct dye, preferably selected form anionic, cationic and neutral nitro dyes.

11. Composition according to any of the preceding claims **characterized in that** it comprises additionally a compound chosen from allantoin, ubiquinone or diamide compounds.

12. Composition according to any of the preceding claims **characterized in that** the composition has a pH in the range of 6 to 12.

13. Process for dyeing hair wherein a composition according to any of the preceding claims is mixed with an aqueous composition comprising an oxidizing agent prior to application, and the mixture is applied onto hair and rinsed off from hair after processing for 1 to 45 min, optionally hair is shampooed and dried.

14. Kit for dyeing hair which comprises an individually packaged first component comprising a composition according to claim 1 to 12 and an individually packaged second aqueous composition comprising an oxidizing agent.

15. Use of the composition according to claim 1 to 12 for eliminating grey shimmering on hair.

## Patentansprüche

1. Wässrige Zusammensetzung zum Färben von Haaren, **dadurch gekennzeichnet, dass** sie mindestens eine Haar-Oxidationsfarbstoff-Vorstufe, mindestens ein kationisches Tensid als einen Emulgator, mindestens ein kationisches Polymer mit einer kationischen Ladungsdichte größer oder gleich 4 meq/g bei einer Gesamtkonzentration von von 0,05 und 2 Gewichts-%, bezogen auf die Gesamtzusammensetzung, mindestens ein Heteropolysaccharid-Gummi aufweist und frei von anionischen Emulgatoren ist.

2. Zusammensetzung nach Anspruch 1, **dadurch gekennzeichnet, dass** die Zusammensetzung eine Öl-in-Wasser-Emulsion ist und Wasser im Bereich von 50 bis 90 Gewichts-%, mehr bevorzugt im Bereich von 55 bis 85 Gewichts-%, am meisten bevorzugt im Bereich von 60 bis 80 Gewichts-% aufweist, bezogen auf die Gesamtzusammensetzung.

3. Zusammensetzung nach Anspruch 1 und 2, **dadurch gekennzeichnet, dass** das mindestens eine kationische Tensid ausgewählt ist aus quartären Ammoniumverbindungen gemäß der allgemeinen Struktur 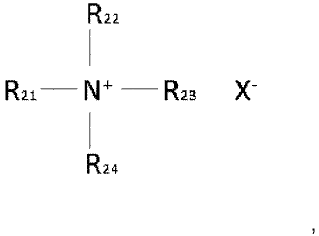 wobei R₂₁ für eine gesättigte oder ungesättigte, verzweigte oder unverzweigte Alkylkette mit 8 bis 22 C-Atomen oder R₂₅CONH(CH₂)ₙ oder R₂₅COO(CH₂)ₙ steht, wobei R₂₅ für eine gesättigte oder ungesättigte, verzweigte oder unverzweigte Alkylkette mit 7 bis 21 C-Atomen steht und n einen typischen Wert von 1 bis 4 aufweist, und R₂₂, R₂₃ und R₂₄ unabhängig voneinander für eine niedere Alkylkette mit 1 bis 4 Kohlenstoffatomen, eine Hydroxyalkylkette mit 1 bis 4 C-Atomen oder eine Ethoxy- oder Propoxygruppe stehen, wobei die Anzahl der Ethoxy- oder Propoxygruppen im Bereich von 1 bis 4 variiert, und X für Chlorid, Bromid oder Methosulfat steht.

4. Zusammensetzung nach einem der vorhergehenden Ansprüche, **dadurch gekennzeichnet, dass** das mindestens eine kationische Polymer mit einer kationischen Ladungsdichte größer oder gleich 4 meq/g aus polymerem Diallyldimethylammoniumchlorid ausgewählt ist.

5. Zusammensetzung nach einem der vorhergehenden Ansprüche, **dadurch gekennzeichnet, dass** das mindestens eine Heteropolysaccharid-Gummi aus Xanthan, Carrageenan, Guaran und Johannisbrotgummi ausgewählt ist, das Heteropolysaccharid-Gummi vorzugsweise aus Xanthan ausgewählt ist und vorzugsweise in einer Konzentration von 0,1 bis 2 Gewichts-% vorliegt, bezogen auf die Gesamtzusammensetzung.

6. Zusammensetzung nach einem der vorhergehenden Ansprüche, **dadurch gekennzeichnet, dass** das mindestens eine kationische Tensid als ein Emulgator in einer Gesamtkonzentration von 0,1 bis 2 Gewichts-% verwendet wird, bezogen auf die Gesamtzusammensetzung.

7. Zusammensetzung nach einem der vorhergehenden Ansprüche, **dadurch gekennzeichnet, dass** das kationische Polymer mit einer kationischen Ladungsdichte größer oder gleich 4 meq/g in einer Gesamtkonzentration von 0,05 bis 1,5 Gewichts-%, mehr bevorzugt 0,1 bis 1 Gewichts-%, verwendet wird, bezogen auf die Gesamtkonzentration.

8. Zusammensetzung nach einem der vorhergehenden Ansprüche, **dadurch gekennzeichnet, dass** sie mindestens ein Alkalisierungsmittel aufweist, ausgewählt aus Ammoniak und Verbindungen der folgenden allgemeinen Struktur
R₁₁R₁₂R₁₃N,
wobei R₁₁, R₁₂ und R₁₃ gleich oder verschieden sind und für H, C₁₋₆-Alkyl, C₁₋₆-Monohydroxyalkyl oder C₂₋₆-Polyhydroxyalkyl stehen, mit der Maßgabe, dass mindestens eines aus R₁₁, R₁₂ und R₁₃ für ein Mono- oder Polyhydroxyalkyl steht.

9. Zusammensetzung nach einem der vorhergehenden Ansprüche, **dadurch gekennzeichnet, dass** das mindestens eine Alkalisierungsmittel aus Alkanolaminen ausgewählt ist, vorzugsweise in einer Konzentration im Bereich von 1 bis 35 Gewichts-%, bezogen auf die Gesamtzusammensetzung.

10. Zusammensetzung nach einem der vorhergehenden Ansprüche, **dadurch gekennzeichnet, dass** sie zusätzlich einen Haardirektfarbstoff aufweist, vorzugsweise ausgewählt aus anionischen, kationischen und neutralen Nitrofarbstoffen.

11. Zusammensetzung nach einem der vorhergehenden Ansprüche, **dadurch gekennzeichnet, dass** sie zusätzlich eine Verbindung aufweist, ausgewählt aus Allantoin, Ubichinon oder Diamidverbindungen.

12. Zusammensetzung nach einem der vorhergehenden Ansprüche, **dadurch gekennzeichnet, dass** die Zusammensetzung einen pH-Wert im Bereich von 6 bis 12 aufweist.

13. Verfahren zum Färben von Haaren, wobei eine Zusammensetzung nach einem der vorhergehenden Ansprüche vor dem Aufbringen mit einer wässrigen Zusammensetzung vermischt wird, welche ein Oxidationsmittel aufweist, und das Gemisch auf die Haare aufgebracht und nach einer Einwirkungszeit von 1 min bis 45 min aus den Haaren ausgespült wird, die Haare gegebenenfalls shampooniert und getrocknet werden.

14. Kit zum Färben von Haaren, welches eine einzeln verpackte erste Komponente, die eine Zusammensetzung nach Anspruch 1 bis 12 aufweist, und eine einzeln verpackte zweite wässrige Zusammensetzung aufweist, die ein Oxidationsmittel aufweist.

15. Verwendung der Zusammensetzung nach Anspruch 1 bis 12 zum Beseitigen eines Grauschimmers an Haaren.

## Revendications

1. Composition aqueuse pour la coloration des cheveux **caractérisée en ce qu'**elle comprend au moins un précurseur de colorant d'oxydation des cheveux, au moins un tensioactif cationique servant d'émulsifiant, au moins un polymère cationique avec une densité de charge cationique égale ou supérieure à 4 meq/g à une concentration totale de 0,05 et 2 % en poids, calculée par rapport au total de la composition, au moins une gomme hétéro polysaccharide et qu'elle est exempte d'émulsifiants anioniques.

2. Composition selon la revendication 1, **caractérisée en ce que** la composition est une émulsion huile dans l'eau et comprend de l'eau dans la plage de 50 à 90 %, plus préférentiellement dans la plage de 55 à 85 %, idéalement dans la plage de 60 à 80 % en poids, calculée par rapport à la composition totale.

3. Composition selon les revendications 1 et 2, **caractérisée en ce que** l'au moins un tensioactif cationique est choisi parmi des composés d'ammonium quaternaires selon la structure générale 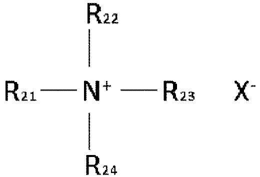 où R₂₁ est une chaîne alkyle saturée ou insaturée, ramifiée ou non ramifiée, avec 8 à 22 atomes C, ou R₂₅CONH(CH₂)n, ou R₂₅COO(CH₂)n, où R₂₅ est une chaîne alkyle saturée ou insaturée, ramifiée ou non ramifiée, avec 7 à 21 atomes C et n possède une valeur typique de 1 à 4, et R₂₂, R₂₃ et R₂₄ sont de manière indépendante les uns des autres une chaîne alkyle courte avec 1 à 4 atomes de carbone, une chaîne hydroxyl alkyl avec 1 à 4 atomes C, ou un groupement éthoxy ou propoxy avec un nombre de groupements éthoxy ou propoxy variant dans la plage de 1 à 4, et X est du chlorure, du bromure ou du méthosulfate.

4. Composition selon l'une quelconque des revendications précédentes, **caractérisée en ce que** l'au moins un polymère cationique avec une densité de charge cationique égale ou supérieure à 4 meq/g est choisi parmi un chlorure d'ammonium diméthyl diallylique polymérique.

5. Composition selon l'une quelconque des revendications précédentes, **caractérisée en ce que** l'au moins une gomme hétéro polysaccharide est choisie parmi la gomme xanthane, les carraghénanes, la gomme de guar et la gomme de graine de lotus, de préférence la gomme hétéro polysaccharide est choisie parmi la gomme xanthane de préférence présente à une concentration de 0,1 à 2 % en poids, calculée par rapport au total de la composition.

6. Composition selon l'une quelconque des revendications précédentes, **caractérisée en ce que** l'au moins un tensioactif cationique servant d'émulsifiant est utilisé à une concentration totale de 0,1 à 2 % en poids, calculée par rapport au total de la composition.

7. Composition selon l'une quelconque des revendications précédentes, **caractérisée en ce que** le polymère cationique avec une densité de charge cationique égale ou supérieure à 4 meq/g est utilisé à une concentration totale de 0,05 à 1,5 %, plus préférentiellement de 0,1 à 1 % en poids, calculée par rapport au total de la composition.

8. Composition selon l'une quelconque des revendications précédentes, **caractérisée en ce qu'**elle comprend au moins un agent alcalinisant choisi parmi l'ammoniac et des composés selon la structure générale suivante
R₁₁R₁₂R₁₃N
où R₁₁, R₁₂ et R₁₃ sont, de manière identique ou différente, l'atome H, un groupement alkyle en C₁ à C₆, un groupement monohydroxy alkyle en C₁ à C₆ ou polyhydroxy alkyle en C₂à C₆, à condition qu'au moins un parmi R₁₁, R₁₂ et R₁₃ soit un groupement mono ou polyhydroxy alkyle.

9. Composition selon l'une quelconque des revendications précédentes, **caractérisée en ce que** l'au moins un agent alcalinisant est choisi parmi des alcanolamines, de préférence à une concentration dans la plage de 1 à 35 % en poids calculée par rapport à la composition totale.

10. Composition selon l'une quelconque des revendications précédentes, **caractérisée en ce qu'**elle comprend en supplément un colorant direct des cheveux, de préférence choisi parmi des colorants anioniques, cationiques et nitro neutres.

11. Composition selon l'une quelconque des revendications précédentes, **caractérisée en ce qu'**elle comprend en supplément un composé choisi parmi des composés d'allantoïne, d'ubiquinone ou des diamides.

12. Composition selon l'une quelconque des revendications précédentes, **caractérisée en ce que** la composition a un pH dans la plage de 6 à 12.

13. Procédé pour la coloration des cheveux dans lequel une composition selon l'une quelconque des revendications précédentes est mélangée avec une composition aqueuse comprenant un agent d'oxydation avant l'application, et le mélange est appliqué sur les cheveux, et est enlevé des cheveux par rinçage après un traitement pendant 1 à 45 min, éventuellement, les cheveux sont shampouinés et séchés.

14. Kit pour la coloration des cheveux qui comprend un premier composant emballé individuellement comprenant une composition selon la revendication 1 à 12 et une seconde composition aqueuse emballée individuellement comprenant un agent d'oxydation.

15. Utilisation de la composition selon la revendication 1 à 12 pour éliminer un reflet gris sur les cheveux.
